# EUROPEAN PATENT APPLICATION

(11) **EP 2 028 264 A1**
(43) Date of publication of application: **25.02.2009**
(21) Application number: 07744610.2
(22) Date of filing: 01.06.2007
(51) Int. Cl.: C12M 3/00, G02B 7/04, G02B 21/00

(54) **CELL INCUBATOR**

(30) Priority: 15.06.2006 JP 2006165898
(71) Applicant: Nikon Corporation, Tokyo 100-8331 (JP)
(72) Inventor: KIYOTA, Yasujiro, Chiyoda-ku, Tokyo 100-8331 (JP); UOZUMI, Takayuki, Chiyoda-ku, Tokyo 100-8331 (JP)
(74) Representative: Cabinet Plasseraud
(86) International application number: PCT/JP2007/061223
(87) International publication number: WO 2007/145091

(57) **Abstract**

A cell incubator (10) having an observation unit (70) to observe a cell incubation process comprises: a focus detecting unit (110) to detect a focus position of an imaging optical system (71) of the observation unit (70); an optimum focus selecting unit (120) to select an optimum focus position based upon a plurality of images taken by moving the imaging optical system (71) stepwise at a fine pitch along an optical axis direction from the focus position, based upon the focus position detected by the focus detecting unit (110); and a fluorescence observation unit (130) to shoot a fluorescence image of the cells by moving the imaging optical system (71) to the optimum focus position thus detected by the optimum focus selecting unit (120).

## Description

### TECHNICAL FIELD

The present invention relates to a cell incubator which incubates cells, and which allows the cells thus incubated to be observed.

### BACKGROUND ART

In prior arts, cell incubators have been proposed that have a storage unit in which cells are incubated, inside a thermostatic chamber maintained at a prescribed atmosphere, and an observation unit to observe a cell incubation process (Patent Document 1, for example).

In cases of detailed observation of micro structure of each organelle such as cell nucleus, mitochondria, etc., the cell incubator disclosed in Patent Document 1 requires a great amount of time to detect optimum focus position. Furthermore, such an arrangement has a problem in that, the fluorescence image acquired in the fluorescence observation mode cannot be clearly and precisely observed in correlation with the organelles to be observed. Moreover, with conventional fluorescence observation, excitation light is emitted to the cells, and focus adjustment is performed based upon the fluorescence emitted from the cells. This leads to adverse effects such as fluorescence discoloration in the focus adjustment, and damage to the cells due to the excitation light.

Patent Document 1 Japanese Patent Application Laid-Open No. 2004-180675

### DISCLOSURE OF THE INVENTION

### Problems to be Solved by the Invention

It is an object of the present invention to provide a cell incubator which allows an image of the cells and a fluorescence image thereof to be acquired at a high speed, and which allows these images to be observed with high precision in correlation with each other.

### Means for Solving the Problems

With the present invention, the aforementioned problems are solved by the following solving means. It should be noted that, in order to facilitate understanding, description will be made using reference numerals in parentheses that correspond to the embodiments according to the present invention. However, the present invention is not restricted to such embodiments.

An aspect according to the invention as disclosed in Claim 1 relates to a cell incubator (10) having an observation unit (70) to observe a cell incubation process. The cell incubator (10) comprises: a focus detecting unit (110) to detect a focus position of an imaging optical system (71) of the observation unit (70); an optimum focus selecting unit (120) to select an optimum focus position based upon a plurality of images taken by moving the imaging optical system (71) stepwise at a fine pitch along an optical axis direction from the focus position, based upon the focus position detected by the focus detecting unit (110); and a fluorescence observation unit (130) to shoot a fluorescence image of the cells by moving the imaging optical system (71) to the optimum focus position thus detected by the optimum focus selecting unit (120).

An aspect according to the invention as disclosed in Claim 2 relates to the cell incubator (10) described in Claim 1, in which the focus detecting unit (110) comprises: a wide area detecting unit (111) to set a wide area extending along an optical axis direction of the imaging optical system (71) to be a focus detecting region, and to detect a focus position over the wide focus detecting region; a restricted detecting unit (112) to set a restricted region, which is narrower than the wide focus detecting area of the wide area detecting unit (111) and which contains the focus position detected by the wide area detecting unit (111), to be a restricted focus detecting region, and to detect a focus position over the restricted focus detecting region; and a detailed detecting unit (113) to set a restricted region, which is narrower than the restricted focus detecting region for the restricted detecting unit (112) and which contains the focus position detected by the restricted detecting unit (112), to be a detailed area focus detecting region, and to detect a focus position over the detailed area focus detecting region.

An aspect according to the invention as disclosed in Claim 3 relates to the cell incubator (10) described in Claim 1 or Claim 2, in which the focus detecting unit (110) detects a focus position by moving the imaging optical system (71) stepwise or continuously, and the optimum focus selecting unit (120) moves the imaging optical system (71) by a smaller movement distance than the focus detecting unit (110) moves the imaging optical system (71) stepwise or continuously.

An aspect according to the invention disclosed in Claim 4 relates to the cell incubator (10) described in any one of Claim 1 through Claim 3. The cell incubator (10) further includes an image processing unit (140) to create a superimposition image by superimposing the fluorescence image acquired by the fluorescence observation unit (130) and the image acquired at the optimum focus position.

An aspect according to the invention as disclosed in Claim 5 relates to a cell incubator (10) having an observation unit (70) to observe a cell incubation state. The cell incubator (10) comprises: a wide area detecting unit (111) to set a wide area extending along an optical axis direction of the observation unit (70) to be a focus detecting region, and to detect a focus position over the wide focus detecting region; a restricted detecting unit (112) to set a restricted region, which is narrower than the wide focus detecting area of the wide area detecting unit (111) and which contains the focus position detected by the wide area detecting unit (111), to be a restricted focus detecting region, and to detect a focus position over the restricted focus detecting region; a detailed detecting unit (113) to set a restricted region, which is narrower than the restricted focus detecting region for the restricted detecting unit (112) and which contains the focus position detected by the restricted detecting unit (112), to be a detailed area focus detecting region, and to detect a focus position over the detailed area focus detecting region; and an image acquisition unit (120) to set a predetermined focus detecting region that contains the focus position detected by the detailed detecting unit (113) and that extends along the optical axis direction, and to acquire a plurality of images while an optical system (71) of the observation unit (70) is moved over the predetermined focus detecting region.

An aspect according to the invention as disclosed in Claim 6 relates to a cell incubator (10) including a stage on which a cell incubation container (15) is mounted, an observation unit (70) which allows a cell incubation state of cells contained in the cell incubation container (15) to be observed, and a moving unit (78) which relatively moves the stage and the observation unit (70). The cell incubator (10) comprises a wide area detecting unit (111) to set a wide area extending along an optical axis direction of the observation unit (70) to be a focus detecting region, and to detect a focus position over the wide focus detecting region; a restricted detecting unit (112) to set a restricted region, which is narrower than the wide focus detecting area of the wide area detecting unit (111) and which contains the focus position detected by the wide area detecting unit (111), to be a restricted focus detecting region, and to detect a focus position over the restricted focus detecting region; a detailed detecting unit (113) to set a restricted region, which is narrower than the restricted focus detecting region for the restricted detecting unit (112) and which contains the focus position detected by the restricted detecting unit (112), to be a detailed area focus detecting region, and to detect a focus position over the detailed area focus detecting region; and an image acquisition unit (120) to set a predetermined focus detecting region that contains the focus position detected by the detailed detecting unit (113) and that extends along the optical axis direction, and to acquire a plurality of images while an optical system (71) of the observation unit (70) is moved over the predetermined focus detecting region; and a control unit (100) to control the moving unit (78) such that it is moved to a different observation point within the cell incubation container (15), and controls the restricted detecting unit (112), the detailed detecting unit (113), and the image acquisition unit (120) such that, when the image of cells is acquired by the image acquisition unit (120) at the observation point after the movement of the observation point, subsequent processing is executed using the focus position detected by the wide area detecting unit (111) before the movement of the observation point, or the focus position detected by the restricted detecting unit (112) before the movement of the observation point.

### Effects of the Invention

As described above, the cell incubator according to the present invention includes: the focus detecting unit to detect the focus position for the imaging optical system; the optimum focus selecting unit which selects the optimum focus position based upon the focus positions; and the fluorescence observation unit which performs fluorescence observation after the imaging optical system is moved to the optimum focus position. Thus, such an arrangement allows the optimum focus position to be detected at a high speed, and allows a clear fluorescence image of cells to be observed with high precision.

### BRIEF DESCRIPTION OF THE DRAWINGS

Fig. 1 is a diagram which shows a cell incubator according to an embodiment of the present invention;
Fig. 2 is a diagram which shows a detailed configuration of an observation unit according to the embodiment of the present invention;
Fig. 3 is a diagram which shows an example of an observation image acquired by the observation unit according to the embodiment of the present invention;
Fig. 4 is a flowchart which shows the method for using the cell incubator according to the embodiment of the present invention; and
Fig. 5 is a diagram for describing the detection start position for an objective lens unit according to the embodiment of the present invention.

### PREFERRED MODE FOR CARRYING OUT THE INVENTION

The present invention achieves the purpose of providing a cell incubator which allows an image of cells and a fluorescent image thereof to be acquired at a high speed, and which allows these images to be observed with high precision in correlation with each other, as follows. First, the cells are observed in a phase contrast observation mode, and focus detecting is performed based upon the image thus observed, thereby obtaining a focus position. Subsequently, image information is acquired over a further narrowed area so as to obtain the optimum focus position. A fluorescence image is acquired at the optimum focus position thus obtained.

A further detailed description will be made below regarding an embodiment according to the present invention with reference to the drawings etc.

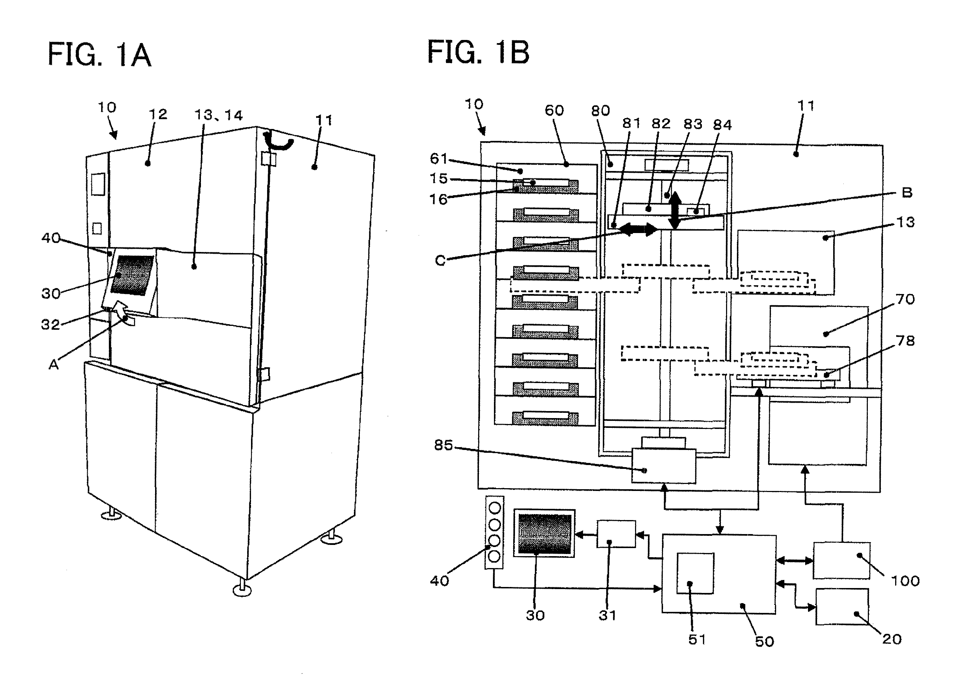

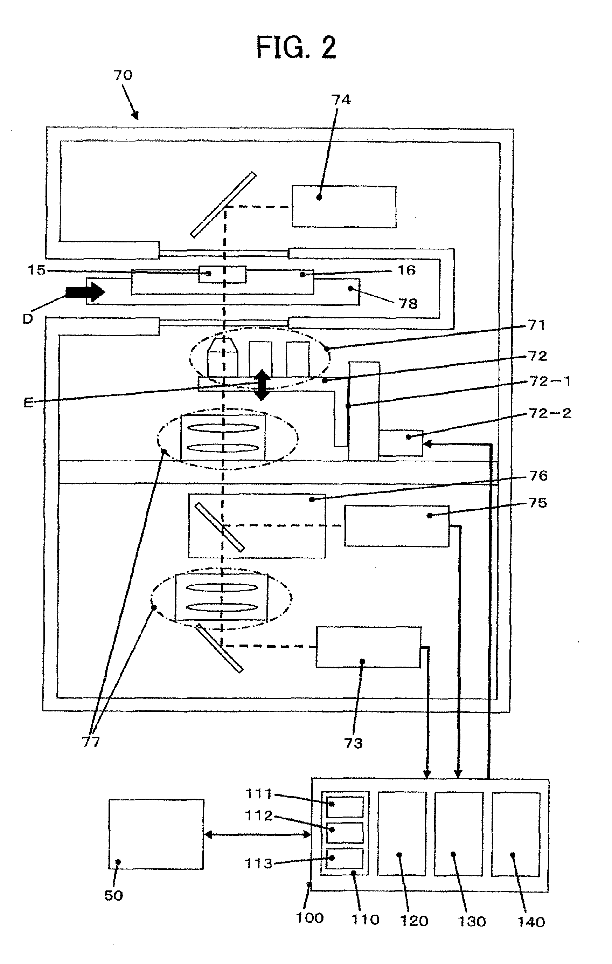

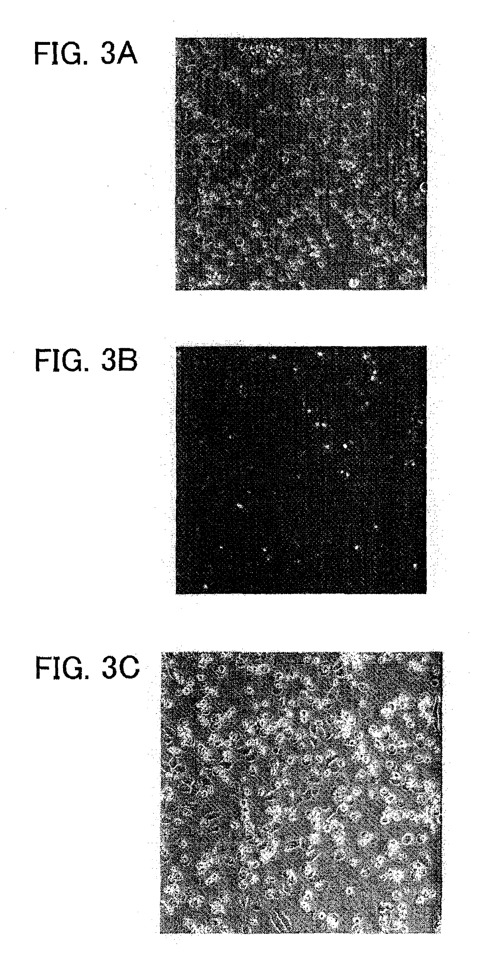

Fig. 1 is a diagram which shows a cell incubator according to an embodiment of the present invention. It should be noted that Fig.1A is an external view of the cell incubator. Fig.1B is a diagram which shows a detailed configuration of the interior of the cell incubator and connections of a control unit. Fig. 2 is a detailed configuration of an observation unit of the cell incubator. Fig. 3 is a diagram which shows an example of images taken by the observation unit. It should be noted that Fig.3A is a view which shows a phase contrast image. Fig.3B is a view which shows a fluorescence image. Fig.3C is a view which shows a superimposition image created by superimposing the fluorescence image and the phase contrast image.

As shown in Fig. 1, a cell incubator 10 is an apparatus including a thermostatic chamber 11, an opening/closing door 12, an inserting/extracting unit 13, an information storage unit 20, an information display unit 30, an operation input unit 40, and a control unit 50. Furthermore, the thermostatic chamber 11 includes a storage unit 60, an observation unit 70, and a conveying unit 80 therewithin. The cell incubator 10 allows a plurality of cells to be incubated, and allows a cell incubation process to be automatically observed.

The thermostatic chamber 11 is a chamber which maintains a constant atmosphere at a temperature of 37°C, a humidity of 97%, and a CO₂ concentration of 5%.

The opening/closing door 12 is a door provided over the entire area of the front face of the thermostatic chamber 11. When maintenance is performed for the storage unit 60, the observation unit 70, and the conveying unit 80, or the like, the opening/closing door 12 is opened/closed. Furthermore, the inserting/extracting unit 13, the information display unit 30, and the operation input unit 40 are arranged on the opening/closing door 12, which improves user operability.

The inserting/extracting unit 13 is an inserting/extracting opening which allows cells to be inserted into/extracted from the thermostatic chamber 11. When there is no cell insertion/extraction, the inserting/extracting unit 13 is closed by means of an inserting/extracting door 14 in order to maintain the atmosphere in the thermostatic chamber 11.

The inserting/extracting door 14 is a door having a locking mechanism opening/closing to inside the thermostatic chamber 11.

A cell incubation container 15 is a container in which cells are incubated. Examples thereof include a dish, a well plate, a flask, etc.

A holder 16 is a holder member which allows the cell incubation container 15 to be properly arranged in each of the container unit 60, the observation unit 70, and the conveying unit 80 included in the cell incubator 10.

As shown in Fig.1B, the information storage unit 20 is memory which stores information with respect to cells, and information with respect to the observation results for the cells observed using the observation unit 70. It should be noted that information with respect to cells is principally information regarding the properties of the cells, such as kind of cells, culture composition information, initial number of cells, culture replacement frequency, successive cultivation period, etc., and includes information associated therewith such as kind of cell incubation container 15, information with respect to a manager who manages the cells, experimental information with respect to the cell cultivation (experiment name), information with respect to the cell cultivation history, etc.

The information display unit 30 is a display which displays information stored in the information storage unit 20, information with respect to operation of the cell incubator 10, etc. The information display unit 30 can be tilted by means of a tilt mechanism 32 so as to facilitate observation by the user, as shown in Fig.1A (arrow A).

A display format control unit 31 is a display control unit which allows the elapsed-time in the individual cell information to be displayed in a simple manner using changes in color.

The operation input unit 40 is an input device which allows the cell information to be input, and which allows operation of the cell incubator 10 to be determined according to the content displayed on the information display unit 30, thereby inputting an operation instruction to the control unit 50. The operation input unit 40 has a configuration including a plurality of buttons.

The control unit 50 is a control circuit which performs control so as to maintain the atmosphere in the thermostatic chamber 11 under a constant environmental conditions, and which integrally controls the observation control unit 100 and the observation unit 70 and the conveying unit 80 included within the thermostatic chamber 11 according to the content of operations input via the operation input unit 40.

The user identification unit 51 is a confirmation circuit provided in the control unit 50. The user identification unit 51 determines whether or not a log-in request for the cell incubator 10 is valid by make user to input an user ID. The cell incubator 10 is managed such that it cannot be operated unless the user inputs a correct password set beforehand. Such an arrangement protects the cell incubator 10 from being operated arbitrarily by unauthorized people. Furthermore, the user ID is associated with the cell manager information with respect to the cells stored in the cell incubator 10. The cell incubator 10 limits operation such that operation thereof is permitted only in a case in which the user ID matches the cell manager information with respect to the cell manager information that manages the cells stored in the information storage unit 20, so as to protect registered cells from mistaken operation by others, in cases in which a plurality of users have been registered.

The storage unit 60 interior is divided into a plurality of storage sections 61. The cell incubation container 15 containing cells is stored in respective storage sections 61 via the holder 16, thereby allowing the cells to be incubated. With the present embodiment, the storage unit 60 has 27 storage sections 61 in the form of a matrix having 9 rows along the vertical direction and 3 columns along the horizontal direction.

As shown in Fig. 2, the observation unit 70 is a microscope which includes an objective lens 71, a Z-axis mechanism unit 72, a CCD camera unit 73, an LED illumination unit 74, a fluorescent illumination unit 75, a fluorescence filter unit 76, a lens unit 77, and an XY stage unit 78. The observation unit 70 allows the cells to be observed (allows an image of the cells to be taken). The observation unit 70 allows the user to observe the state of each organelle such as nucleus, mitochondria, etc., in fluorescence observation mode (molecular observation mode), in addition to observation of cells in phase contrast observation mode.

The objective lens unit 71 has a configuration including a plurality of objective lenses, which can be separately selected based upon kind of cell, observation area, etc.

The Z-axis mechanism unit 72 includes a guide mechanism 72-1 and a driving motor 72-2. The Z-axis mechanism unit 72 is a driving mechanism which adjusts focus by moving the objective lens unit 71 along the vertical direction (Z-axis direction) by a small movement distance.

The CCD camera unit 73 is a camera which takes an ordinary observation image (phase contrast image) and a fluorescence observation image (fluorescence image) of the cells contained in the cell incubation container 15.

The LED illumination unit 74 is an illumination unit employing LEDs, which illuminates the cells contained in the cell incubation container 15. The LED illumination unit 74 emits illumination light for image taking, synchronously with the exposure time for the CCD camera unit 73. Furthermore, the LED illumination unit 74 intermittently emits illumination light with movement of the Z-axis mechanism unit 72 in focus detection.

The fluorescence illumination unit 75 is an illumination unit which emits excitation light which excites fluorescent protein of the cells, thereby allowing the organelles of the cells to be observed.

The fluorescence filter unit 76 has a configuration including a wavelength-selectable dichroic mirror and a bandpass filter. Such an arrangement allows the CCD camera unit 73 to take an image of the fluorescent protein of the cells and which has been excited by the excitation light emitted from the fluorescence illumination unit 75 to the entire area along the optical axis direction.

The XY stage unit 78 is a precision stage which allows the cell incubation container 15, which has been conveyed by the conveying unit 80, to be movable to a desired position along a horizontal direction (XY direction). Such an arrangement allows the cell incubation container 15 to be moved to an observation position of the observation unit 70.

The conveying unit 80 includes a conveying arm 81, a moving member 82, a screw shaft 83, an arm motor 84, and a moving motor 85. The conveying unit 80 is a conveying mechanism which conveys the cell incubation container 15 with the holder 16 to the inserting/extracting unit 12, the storage unit 60, or the observation unit 70, within the thermostatic chamber 11. In the conveying unit 80, the moving member 82, which meshes with a screw shaft 83 which has a helical groove formed in the face thereof, is driven along the vertical direction (arrow B) by the moving motor 85. Furthermore, the conveying arm 81 supported by the moving member 82 is moved along the horizontal direction (arrow C) by the arm motor 84. Thus, such an arrangement allows the cell incubation container 15 to be received from/delivered to the inserting/extracting unit 13, the storage unit 60 or the observation unit 70. Furthermore, the screw shaft 83 includes a mechanism (not shown) which allows movement along a direction orthogonal to the drawing in Fig. 1 (horizontal direction).

The observation control unit 100 includes a focus detecting unit 110, an optimum focus selecting unit 120, a fluorescence observation unit 130, and an image processing unit 140. The observation control unit 100 is a controller which integrally controls the observation unit 70 according to an instruction signal received from the operation input unit 40 and the control unit 50.

The focus detecting unit 110 includes a wide area detecting unit 111, a restricted detecting unit 112, and a detailed detecting unit 113. The focus detecting unit 110 is a circuit which automatically detects the focus position (automatic focusing) based upon the images of the CCD camera unit 73 with the objective lens unit 71 moving up/down by means of the Z-axis mechanism unit 72. In order to reduce the focus detecting time, the optimum initial position of the objective lens unit 71 can be computed giving consideration to the kind of cells, the thickness of the bottom of the cell incubation container 15, etc., based upon cell information, information with respect to the kind of cell incubation container 15, etc., read from the information storage unit 20. Furthermore, the objective lens unit 71 can be moved to the initial position thus computed. It should be noted that the focus position is computed as follows. That is, the maximum contrast value and the minimum contrast value are extracted from the pixel values of each image, and the focus position is set to the position that exhibits the maximum value calculated based upon the contrast curve thus acquired (maximum/minimum method). Alternatively, the differences in the image data between adjacent pixels are calculated for each line of the contrast image thus acquired. The sum of the absolute values of the differences thus calculated is calculated for each image thus acquired. The position that corresponds to the maximum value of the absolute sum is set to the focus position (Σ|Δ|method).

The wide area detecting unit 111 performs a focus detecting operation (wide area scanning operation) by driving the objective lens unit 71 over a wide area along the Z-axis direction, i.e., the optical axis direction of the objective lens unit 71. Specifically, the wide area detecting unit 111 performs a focus detecting operation by driving the objective lens unit 71 stepwise at a rough pitch with regard to the focal depth of the objective lens unit 71, or by continuously driving the objective lens unit 71.

The restricted detecting unit 112 performs a focus detecting operation (restricted area scanning operation) by driving the objective lens unit 71 over a restricted area narrower that used by the wide area detecting unit 111. Specifically, the restricted detecting unit 112 sets the focus detecting area to a restricted area which contains the focus position of the objective lens unit 71 obtained by the wide area detecting unit 111. Furthermore, the restricted detecting unit 112 performs a focus detecting operation by driving the objective lens unit 71 stepwise at a moderately rough pitch with regard to the focal depth of the objective lens unit 71, or by continuously driving the objective lens unit 71.

The detailed detecting unit 113 performs a focus detecting operation (detailed scanning operation) by driving the objective lens unit 71 over a more restricted area than that used by the restricted detecting unit 12. Specifically, the detailed detecting unit 113 sets the focus detecting area to a restricted area which contains the focus position of the objective lens unit 71 obtained by the restricted detecting unit 112. Furthermore, the detailed detecting unit 113 performs a focus detecting operation by driving the objective lens unit 71 stepwise at a fine pitch with regard to the focal depth of the objective lens unit 71, or by continuously driving the objective lens unit 71.

As described above, the focus detecting unit 110 includes the wide area detecting unit 111, the restricted detecting unit 112, and the detailed detecting unit 113. Such an arrangement provides three-step focus detecting, thereby detecting the focus position with high precision.

The optimum focus selecting unit 120 identifies the optimum focus position for each of the detecting units 111 through 113 of the focus detecting unit 110. For example, the optimum focus selecting unit 120 acquires a plurality of focus detecting signals by the wide area scanning operation performed by the wide area detecting unit 111. The optimum focus selecting unit 120 relatively evaluates each focus detecting signal so as to extract the optimum focus detecting signal. The focus position obtained based upon the focus detecting signal thus extracted is determined as the optimum focus position by the optimum focus selecting unit 120. In the same way, the optimum focus selecting unit 120 determines the optimum focus position for the restricted detecting unit 112 and the detailed detecting unit 113. The optimum focus positions form references used for determining the focus detecting regions over which the aforementioned detecting units 111 through 113 perform the scanning driving operation.

Based upon the optimum focus position with respect to the detailed detecting unit 113, which was ultimately obtained by the optimum focus selecting unit 120, the CCD camera 73 executes acquisition of a phase contrast image. The CCD camera unit 73 acquires a phase contrast image as shown in Fig.3A.

Specifically, when the optimum focus position is detected by the detailed detecting unit 113, the objective lens unit 71 is controlled and driven stepwise in a Z-axis direction, using the optimum focus position as reference, the CCD camera unit 73 acquires phase contrast images for each stepwise driving operation, to be stored in the information storage unit 20. The stepping movement distance of the stepwise driving operation is set to a smaller movement distance than that used by the detailed detecting unit 113. Also, the optimum focus position is determined based upon the contrast values of the images in the same way as in the focus detecting unit 110.

The fluorescence observation unit 130 is a circuit which instructs the fluorescence illumination unit 75 to excite the fluorescent protein in the cells, and which instructs the CCD camera unit 73 to take a fluorescence image via the fluorescence filter unit 76. It should be noted that the fluorescence image is taken after the objective lens unit 71 is moved to the optimum focus position determined by the optimum focus selecting unit 120.

The image processing unit 140 is an image processing circuit which creates a superimposed image by superimposing the fluorescence image acquired by the fluorescence observation unit 130 and the phase contrast image taken at the optimum focus position selected by the optimum focus selecting unit 120. As shown in Fig.3B, only the points that emit fluorescence by the fluorescence illumination are displayed in the image. With such an arrangement, a superimposition image as shown in Fig.3C is created by superimposing the fluorescence image on the phase contrast image shown in Fig.3A. Such an arrangement allows the user to easily identify the cells and the organelles that emit fluorescence.

Next, a description will be given regarding a method for using the cell incubator 10.

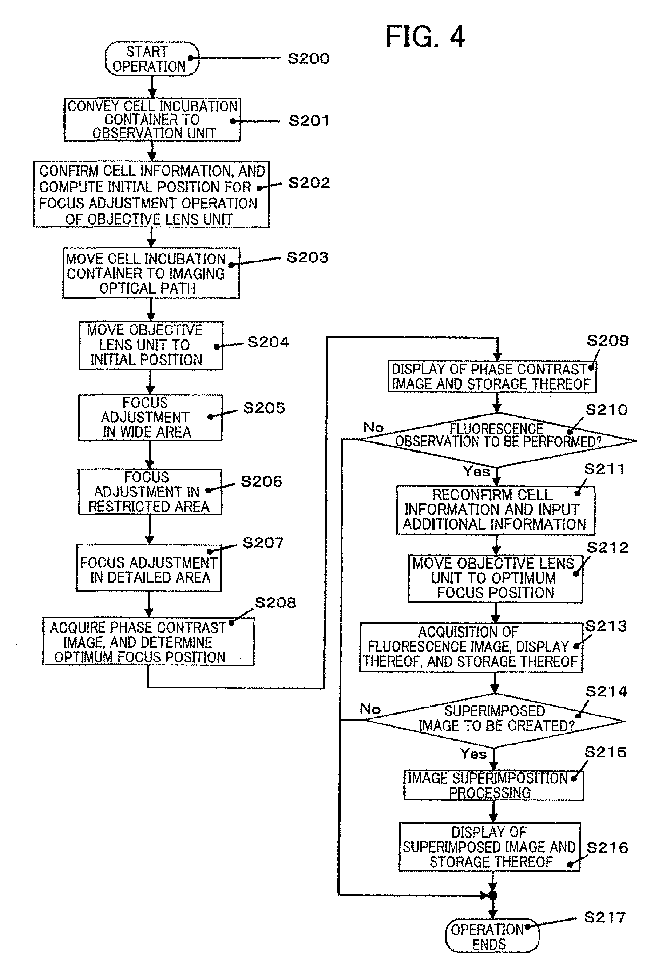

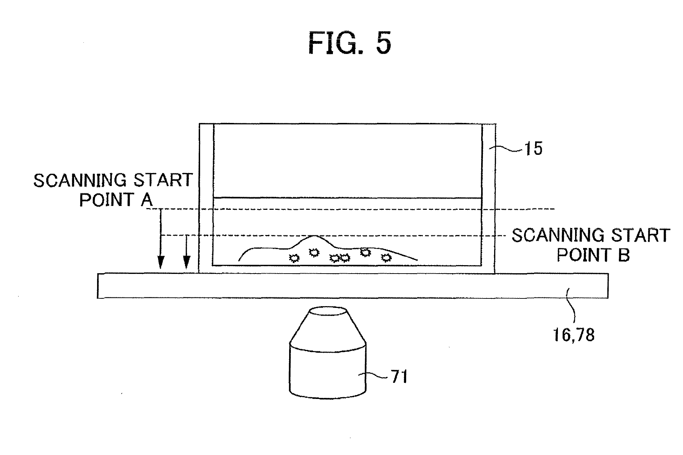

Fig. 4 is a flowchart which shows the method for using the cell incubator. Fig. 5 is a diagram for describing the detecting start position of the objective lens unit of the cell incubator.

When the cell observation operation is started (S200), the cell incubator 10 conveys the cell incubation container 15 from the storage unit 60 or the inserting/extracting unit 13 to the XY stage unit 78 of the observation unit 70 by means of the conveying unit 80 (S201). Furthermore, the cell incubator 10 reads the information with respect to the cells and the cell incubator 15 from the information storage unit 20, and confirms the information thus read out. Moreover, based upon the information thus read, the cell incubator 10 computes the initial position of the objective lens unit 71 of the observation unit 70 for detecting the focus (S202). Subsequently, as shown in Fig. 2, the XY stage unit 78 moves the cell incubation container 15 on the imaging optical path (S203, arrow D). Furthermore, the Z-axis mechanism unit 72 moves the objective lens unit 71 to the initial position thus computed (S204, arrow E).

After the objective lens unit 71 is moved to the initial position, the wide area detecting unit 111 detects the focus position for the objective lens unit 71 over a wide area of the focus detecting region. Here, the initial position of the objective lens unit 71 is determined giving consideration to the thickness of the bottom of the cell incubation container 15 mounted on the XY stage unit 78. For example, as shown in Fig. 5, the wide area detecting unit 111 performs a focus adjustment operation downward from the scanning start point A positioned 7 mm up from the bottom.

Subsequently, the restricted detecting unit 112 detects the focus position thus detected by the wide area detecting unit 111 over the restricted area thus restricted (S206). Furthermore, the detailed detecting unit 113 detects the focus position thus detected by the restricted detecting unit 112 over the detailed region that is more restricted than the restricted region. Thus, the focus detecting unit 110 obtains the focus position (S207). Here, the detecting start positions for the restricted detecting unit 112 and the detailed detecting unit 113 are set to lower positions stepwise, which are lower than the start position (initial position) of the wide area detecting unit 111 described above. For example, the detecting start position for the restricted detecting unit 112 is set to a scanning start point B as shown in Fig. 5, which is set at a position below the detecting start position of the wide area detecting unit 111.

After the focus position is detected by the focus detecting unit 110, the focus position thus detected is set to the base position. Then, phase contrast images (Fig.3A) are taken for each step movement while the objective lens unit 71 is moved stepwise at a fine pitch in the vertical direction (focal direction) from the reference position. Furthermore, the optimum focus selecting unit 120 selects the optimum focus position (S208). The phase contrast image thus taken is displayed on the information display unit 30, and stored in the information storage unit 20 as observation information (S209).

Next, confirmation is made as to whether or not fluorescence observation is to be performed (S210). In a case in which the fluorescence observation is to be performed (Yes in S210), the cell information stored in the information storage unit 20 is reconfirmed. As necessary, additional information necessary for the fluorescence observation is input via the operation input unit 40 (S211). After the reconfirmation and input operation, the objective lens unit 71 is moved to the optimum focus position by means of the Z-axis mechanism unit 72 (S212). Then, the fluorescence observation unit 130 takes a fluorescence image (Fig.3B). The fluorescence image thus acquired is displayed on the information display unit 30, and is stored in the information storage unit 20 (S213). Next, confirmation is made as to whether or not superimposition processing for creating a superimposed image (Fig.3C) is to be performed (S214).

In a case in which the superimposition processing is to be performed (Yes in S214), image processing is performed in which the fluorescence image is superimposed on the phase contrast image (S215). Then, the superimposition image (Fig.3C) is displayed on the information display unit 30, and is stored in the information storage unit 20 (S216). After the storage of the superimposition image, the operation ends (S217).

On the other hand, in a case in which the fluorescence observation is not to be performed (No in S210), or in a case in which the superimposition processing is not to be performed (No in S214), the operation ends without involving the subsequent processing (S217).

With such an arrangement, a plurality of cell incubation containers 15 stored in the storage unit 60 are associated with individual identification numbers (ID numbers). Furthermore, the optimum focus positions used in the first observation are stored in the information storage unit 20. Such an arrangement allows the optimum focus position to be detected quickly in the next observation based upon the focus position thus stored. In particular, such an arrangement provides cell observation with high efficiency and high speed for cases in which there is a need to perform periodic observation, etc.

Next, a description will be given regarding a case of cell observation by the cell incubator 15 in which a plurality of observation points are observed.

With respect to a first observation point, the optimum focus position is obtained according to the control operation as described above with reference in Fig. 4, and a plurality of phase contrast images and fluorescence images are acquired at the optimum focus position, to be stored in the information storage unit 20.

When the observation point is moved from the first observation point to the second observation point, the optimum focus position is obtained as follows, thereby improving operability. That is, the next focus detecting is performed (by the restricted detecting unit 112 or the detailed detecting unit 113) based upon the optimum focus position obtained at the first observation point by means of the wide area detecting unit 111 or the restricted detecting unit 112. Such an arrangement improves the operability. In this step, the processing in Step S206 shown in Fig. 4 or the processing following Step S207 is executed.

As described above, the cell incubator 10 according to the embodiment includes: the focus detecting unit 110 to detect the focus position for the objective lens unit 71; the optimum focus selecting unit 120 which selects the optimum focus position based upon the focus positions; and the fluorescence observation unit 130 which performs fluorescence observation after the objective lens unit 71 is moved to the optimum focus position. Thus, such an arrangement allows the optimum focus position to be detected at a high speed, and allows a clear fluorescence image of cells to be observed with high precision.

## Claims

1. A cell incubator having an observation unit to observe a cell incubation process, the incubator comprising:
a focus detecting unit to detect a focus position of an imaging optical system of the observation unit;
an optimum focus selecting unit to select an optimum focus position based upon a plurality of images taken by moving the imaging optical system stepwise at a fine pitch along an optical axis direction from the focus position, based upon the focus position detected by the focus detecting unit; and
a fluorescence observation unit to shoot a fluorescence image of the cells by moving the imaging optical system to the optimum focus position thus detected by the optimum focus selecting unit.

2. The cell incubator according to Claim 1, wherein
the focus detecting unit comprises:
a wide area detecting unit to set a wide area extending along the optical axis direction of the imaging optical system to be a focus detecting region, and to detect a focus position over the wide focus detecting region;
a restricted detecting unit to set a restricted region, which is narrower than the wide focus detecting area of the wide area detecting unit and which contains the focus position detected by the wide area detecting unit, to be a restricted focus detecting region, and to detect a focus position over the restricted focus detecting region; and
a detailed detecting unit to set a restricted region, which is narrower than the restricted focus detecting region for the restricted detecting unit and which contains the focus position detected by the restricted detecting unit, to be a detailed area focus detecting region, and to detect a focus position over the detailed area focus detecting region.

3. The cell incubator according to Claim 1 or 2, wherein
the focus detecting unit detects each focus position by moving the imaging optical system stepwise or continuously,
and wherein the optimum focus selecting unit moves the imaging optical system by a smaller movement distance than the focus detecting unit moves the imaging optical system stepwise or continuously.

4. The cell incubator according to any one of Claim 1 through Claim 3, further including
an image processing unit to create a superimposition image by superimposing the fluorescence image acquired by the fluorescence observation unit and the image acquired at the optimum focus position.

5. A cell incubator having an observation unit to observe the cell incubation state, comprising:
a wide area detecting unit to set a wide area extending along the optical axis direction of the observation unit to be a focus detecting region, and to detect a focus position over the wide focus detecting region;
a restricted detecting unit to set a restricted region, which is narrower than the wide focus detecting area of the wide area detecting unit and which contains the focus position detected by the wide area detecting unit, to be a restricted focus detecting region, and to detect a focus position over the restricted focus detecting region;
a detailed detecting unit to set a restricted region, which is narrower than the restricted focus detecting region for the restricted detecting unit and which contains the focus position detected by the restricted detecting unit, to be a detailed area focus detecting region, and to detect a focus position over the detailed area focus detecting region; and
an image acquisition unit to set a predetermined focus detecting region that contains the focus position detected by the detailed detecting unit and that extends along the optical axis direction, and to acquire a plurality of images while an optical system of the observation unit is moved over the predetermined focus detecting region.

6. A cell incubator including a stage on which a cell incubation container is to be mounted, an observation unit to observe the cell incubation state of cells contained in the cell incubation container, and a moving device which relatively moves the stage and the observation unit, the incubator comprising;
a wide area detecting unit to set a wide area extending along an optical axis direction of the observation unit to be a focus detecting region, and to detect a focus position over the wide focus detecting region;
a restricted detecting unit to set a restricted region, which is narrower than the wide focus detecting area of the wide area detecting unit and which contains the focus position detected by the wide area detecting unit, to be a restricted focus detecting region, and to detect a focus position over the restricted focus detecting region;
a detailed detecting unit to set a restricted region, which is narrower than the restricted focus detecting region for the restricted detecting unit and which contains the focus position detected by the restricted detecting unit, to be a detailed area focus detecting region, and to detect a focus position over the detailed area focus detecting region;
an image acquisition unit to set a predetermined focus detecting region that contains the focus position detected by the detailed detecting unit and that extends along the optical axis direction, and to acquire a plurality of images while an optical system of the observation unit is moved over the predetermined focus detecting region; and
a control unit to control the moving unit such that it is moved to a different observation point within the cell incubation container, and controls the restricted detecting unit, the detailed detecting unit, and the image acquisition unit such that, when the image of cells is acquired by the image acquisition unit at the observation point after the movement of the observation point, subsequent processing is executed using the focus position detected by the wide area detecting unit before the movement of the observation point, or the focus position detected by the restricted detecting unit before the movement of the observation point.
